# EUROPEAN PATENT APPLICATION

(11) **EP 3 711 847 A1**
(43) Date of publication of application: **23.09.2020**
(21) Application number: 18878929.1
(22) Date of filing: 18.10.2018
(51) Int. Cl.: B01D 53/52, B01D 53/14, C07C 7/11, B01D 53/26, F02B 43/00

(54) **UNIT FOR REFINING BIOGAS AND SUPPLYING BIOMETHANE**

(30) Priority: 18.11.2017 BR 102017024793
(71) Applicant: ER-BR - Energias Renováveis Ltda., Londrina - PR 86042-280 (BR)
(72) Inventor: REINERT, Mário, 86042-280 Londrina (BR); JUNIOR, Cícero, 86042-280 Londrina (BR)
(74) Representative: Nony
(86) International application number: PCT/BR2018/050384
(87) International publication number: WO 2019/095028

(57) **Abstract**

The invention relates to an integrated set of equipment and a process for treating and refining highly contaminated biogas for the purpose of producing biomethane and to the supply thereof in small volumes of up to 10 m3/h to vehicles, such that the refining unit produces small quantities of biomethane per day to supply motor vehicles operating on the same premises as those where the biogas is produced, such as rural properties and small effluent treatment stations. The unit for refining biogas and supplying biomethane, referred to herein as a "biomethane microstation", is an innovative technological concept wherein the microstation comprises the generation of the fuel and the distribution thereof for consumption on the same premises, using a compact biogas refining unit.

## Description

The invention relates to an integrated set of equipment and a process for treating and refining highly contaminated biogas for the purpose of producing biomethane and to the supply thereof in small volumes of up to 10 m3/h to vehicles. The refining unit produces small quantities of biomethane per day to supply motor vehicles operating on the same premises as those where the biogas is produced, such as rural properties and small effluent treatment stations. The unit for refining biogas and supplying biomethane, which is the object of the invention and referred to herein as a "biomethane microstation", is an innovative technological concept. The microstation comprises generation of fuel and distribution thereof for consumption on the same premises.

The biomethane microstation is grounded in the concepts recommended by CIGRE- Conseil Internacional de Grands Reseaux for "microgrids" seen as the necessary path for electricity distribution in the future. In other words, the biomethane microstation is a concept contrary to the main current market trend, which is biomethane gas stations having the same design as liquid fuel or CNG stations. Such stations require investments in transport logistics, either for tank trucks or gas pipelines. The biomethane microstation fills a gap in a new market that the main current market technology fails to meet. As such, the patent application described herein reveals an innovative technology.

Finding a replacement for fossil as a source of electricity and fuel in the global energy supply constitutes a major challenge for society, that has resulted in an intense search for renewable sources of energy. This cannot take long to occur as there is a risk for a world-wide collapse under the current model of development, where oil, coal and nuclear resources are still of great importance.

Another equally important challenge is the search for environmentally viable solutions to eliminate waste produced by human activities and their means of production.

In these scenarios, the use of combustible gases of "renewable origin" can be highlighted as an alternative source of energy. In particular, biogas and biomethane, which are obtained through the biodigestion of organic residues, such as:
Animal waste (pigs, cattle, poultry, etc.);
Vinasse, bagasse and sugarcane leaves;
Urban organic waste (landfills and sewage treatment plants);
Food/agroindustry production waste;
Agricultural and/or forestry waste.

The culture of biogas energy utilization is already stablished. In most of these activities, the use of a portion of the available biogas could be refined and used for self-consumption in the form of vehicle fueling. The use as energy (electric, thermal and vehicular) of these gases requires a high standard of quality, where the removal of all contaminating elements and/or considered harmful to the calorific power is compulsory and regulated by a dedicated legislation.

Among the harmful elements found in biogas we can highlight: CO₂ -carbon dioxide, H₂S-hydrogen sulfide gas, as well as siloxanes. These are the contaminants that hinder the use of biogas and biomethane in combustion engines. Especially in engines for vehicular application, because of the damage this can cause to the engines and thus the release of contaminants generated by their combustion into the atmosphere.

The Brazilian National Oil, Natural Gas and Biofuels Agency - ANP, aiming to fulfill the target and regulations established for the production of experimental fuels, and after a thorough evaluation of the technologies available in the market for biogas purification and with the objective of producing a fuel called biomethane, energetically equivalent to Natural Gas, published the resolution ANP No. 8 of 01/30/2015, which establishes qualitative parameters and the possible applications for Biomethane, as follows:

### 1. ".... Section I-Preliminary provisions

Art. 1 The Biomethane specification contained in the technical regulation by ANP No. 1/2015 is established and integral to this resolution.

Sole paragraph. This resolution is valid to biomethane originating from organic and commercial agrosilvopastoral products and waste for vehicular use (CNG) as well as residential and commercial installations.

The process for biogas refining and biomethane production as described herein promotes greater reach and access to biomethane fuel, with the same technological status as Compressed Natural Gas and meets all ANP regulatory requirements, while achieving biomethane mobility because it supplies biomethane where biogas is produced, specifically to supply the fleet of vehicles operating there.

The challenge is that 100% of the contaminants present in the biogas (CO₂, H₂S, Siloxanes, particulates, humidity, etc.) must be separated in order to produce a high quality biomethane that meets the standards of resolution ANP 08/15.

The process for biogas refining and biomethane production as described herein is related to treatment and biogas refining in small quantities, or flows, in the same place where biogas production occurs, so that the biomethane produced is used for mobility of the local fleet of transport vehicles for persons and materials, as well as agricultural equipment.

Below, patent documents of the current state of the art describe processes that are used in the separation of specific contaminants present in biogas. These industrial processes of biogas refining result in the individual removal of one of the contaminants present in biogas (CO₂, H₂S, Siloxanes, particulates, moisture, etc.). The biomethane produced must undergo several different industrial processes in order to meet the standards of resolution ANP 08/15. The published patent documents are:
A green patent application with registration number BR 102013010204-0: presents high efficiency with total removal of CO₂ carbon dioxide, its application being focused on the production of BIOMETHANE and its biogas presenting low concentration of H₂S Hydrogen Sulfide Gas
A green patent application in progress BR102017001342-1: presents high efficiency in the treatment of acidic gases having high concentration of H₂S- hydrogen sulfide gas. High concentrations (above 1%) are common in biogas produced with livestock waste, mainly for swine farming which is the activity with the highest potential of biogas production;
A green patent application in progress BR 102017017108-6: presents high efficiency in the treatment of gases contaminated with cyclical and linear SILOXANES, the presence of which, even in small quantities causing serious damage to combustion engines. This type of contaminant is common in biogas produced in sewage treatment plants, mainly in urban areas.

The biogas refining unit revealed in this report results in the production of biomethane that provides the removal of 100% of contaminants present in biogas, regardless of the source of biogas.

The refining unit revealed herein, in addition to providing use of biomethane without logistical costs, also does so without intermediation costs, contrary to what is required or inherent to a necessarily centralized large-scale distribution, which burdens the consumer market with an almost absolute paradigm, that is, viability for large-scale businesses only. A situation wherein biomethane does not necessarily need to be limited. Large scale fuel supply is an important path, it must be made feasible and effected, but it is definitely not the only one.

This concept also allows for access to the field of mobility technologies that have long been available, but finding restrictions on feasible availability of biomethane. Such is the case of tractors and other agricultural machinery. For such equipment, the feasibility of supplying them through centralized biogas in refueling stations or logistically moved compressed gas in cylinders is minimal. On the other hand, the biomethane microstation makes it feasible that vehicles can be supplied in rural property and this can only happen if the refining of biogas is carried out by compact units of refining and supply placed along the sources of combustible gases of "renewable origin". This is the function of the refining unit, said "biomethane microstation". It is intended for processing volumes of 1 to 10 m3 per hour, enough to supply 1 to 9 biomethane-powered vehicles per day. Each vehicle with an estimated autonomy between 150 and 200 km/day.

The attached drawings and the detailed description that follows are merely presented as an example, because said "biomethane microstation" can be conceived through other known engineering solutions. Therefore, specific structural and functional details disclosed herein should not be interpreted as a limitation, but only as a basis for the claims; acting as a representative basis for teaching the person skilled in the art to employ and realize the development of the "biomethane microstation", described herein, based on the constructive disposition henceforth detailed.

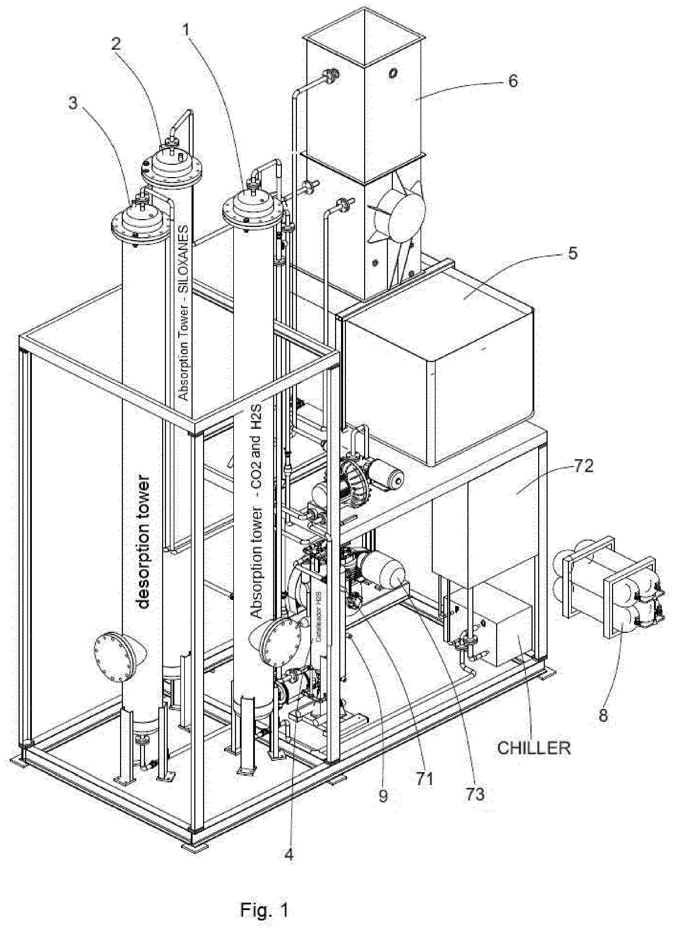

Figure 1 shows a perspective of the "biomethane microstation"

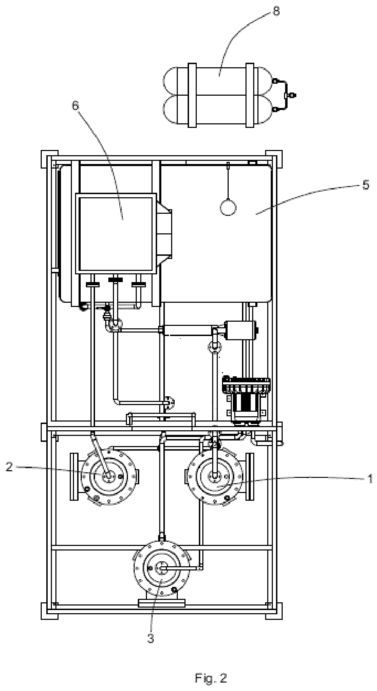

Figure 2 shows the "biomethane microstation" in a floor plan.

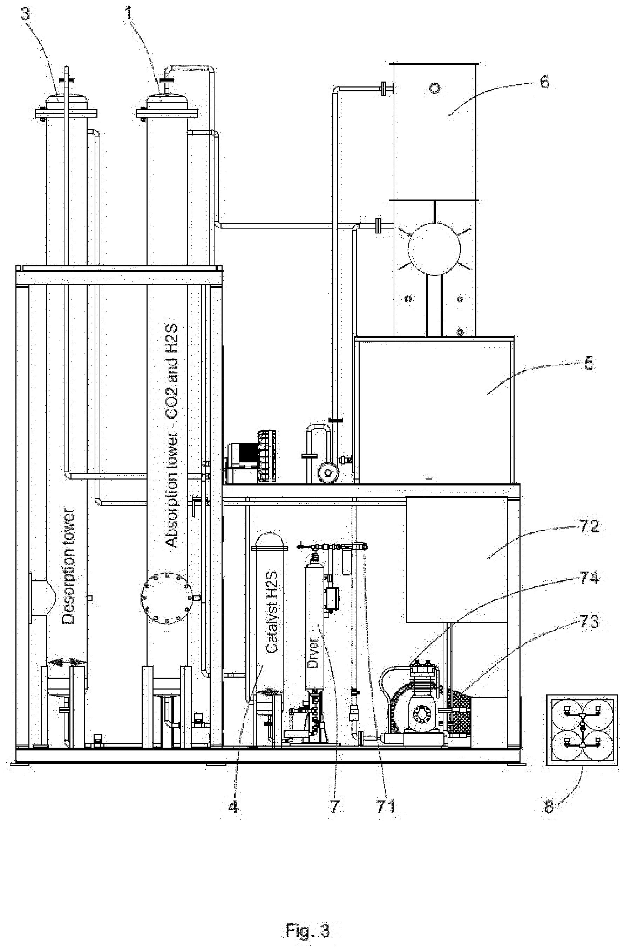

Figure 3 shows a top view of the "biomethane microstation"

The biogas refining and biomethane production unit described herein is characterized by having a CO₂ and H₂S absorption tower (01), a siloxane absorption tower (02), a desorption tower (03), an oxy-catalyst adsorption tower (04), a water tank (05), a cooling tower (06), a cooling tower (06), a biomethane dryer (07), a biomethane outlet (71), a CO₂, H₂S and siloxane outlet (09), an electrical panel (72), a biogas compressor (73)

The biogas refining and biomethane production unit is characterized in that it comprises the following process steps:
1-. BIOGAS for the process is available at the biogas inlet (74), enters the biogas compressor (71) and is compressed according to its composition, with a stabilized pressure within the range of 1 to 35 bar, which ensures high efficiency in all subsequent steps of the process.
2-. Pressurized BIOGAS is sent to cooling tower (06) where the temperature of the water used in the process and the temperature of the gas are calibrated according to its composition. The stabilized temperatures, within the range of 1° to 30° Centigrade, guarantee high efficiency in all stages of the process.
3-. The gaseous compound (biogas) is sent to the CO₂ and H₂S absorption tower (01), where the removal of CO₂-carbon dioxide and H₂S- hydrogen sulfide gas occurs, using only water as a solvent. It results in biomethane.
4-. Only aiming to ensure the total safety of the process in a critical mission analysis, BIOMETHANE is sent to the adsorption tower with Oxy-catalyst (04), whose objective is to prevent the passage of any trace of H₂S-hydrogen sulfide gas not detected by the operating sensors.

The output of the adsorption tower biomethane is monitored by sensors that detect the presence of hydrogen sulfide gas. When a level greater than 0% is found, the process will be interrupted in order to adopt corrective measures.
5-. BIOMETHANE is sent to the SILOXANE Absorption Tower (02) where SILOXANE removal occurs. The SILOXANE absorption Tower (02) will only be installed in the unit when BIOMETHANE is contaminated with this agent;
6-. BIOMETHANE is sent to a dryer (7) to remove excess moisture;
7-. BIOMETHANE is pressurized and stored according to all safety standards, in storage tanks (08), and is ready for use in motor vehicles;
8-. BIOMETHANE is supplied in vehicles using the same technologies available for supply of vehicles with CNG compressed natural gas.

In the first phase of the process, listed above, the biogas compression is calculated according to its composition. The composition of biogas available for the process has several contaminants (CO₂, H₂S, siloxanes, particulates, moisture, etc.) that vary according to their origin. Said origin may be livestock activities, with emphasis on swine farming, milk and beef cattle, animal manure, vinasse, bagasse and sugarcane leaves, landfills and sewage treatment plants, food/agroindustrial, agricultural and/or forestry production waste. According to its biogas composition, the stabilized pressure of biogas is in the range of 1 to 35 bar.

In the second phase of the process above, the biogas temperature is calculated according to its composition, wherein the temperature of the cooling water used in the process and the temperature of the biogas are stabilized within the range of 1° to 30° centigrade

At the end of the treatment and refining stages, all contaminants are removed, in particular CO₂, H₂S, and SILOXANES released at the O₂, H₂S and Siloxane outlet (09), to be processed and economically appraised for industrial applications.

The water used in the process, stored in the water tank (05), is continually regenerated and its replacement is minimal, caused by natural evaporation.

All stages of the process are controlled by an electrical panel (72), a set of sensors that are monitored by a supervisory software, in the occurrence of non-conformity, in case of distortion in the established parameters, the process is stopped until the problem is solved.

Biomethane generated in the microstation has industrial application, having as its target public:
AGRICULTURE: Farmers who have biodigesters installed in their properties and already produce biogas, burn it, or use it for electricity generation. They may be, or intending to employ small volumes of this biogas to supply light vehicles for personal transport, as well as dedicated fleets such as animal transport, fodder distributors, milk collectors and agricultural equipment, tractors, harvesters, self-propelled sprayers and other equipment used in rural areas;
URBAN CENTERS: It has application in activities that produce biodigestible organic solid waste or have easy access to them. Also in locations presenting additional costs to be employed correctly; such as restaurants, hotels, hospitals, industrial kitchens, among other activities. Especially if they have dedicated fleets for transportation of personnel and goods (delivery) presenting ideal space conditions for the installation of biodigesters and the microstation;
Private or public sewage treatment plants: small-sized treatment plants, that produce biogas in small quantities in their proceedings, can provide biomethane for the supply of small fleets (cars, ambulances, etc.).

This biogas refining and biomethane supply unit in a microstation was developed in synergy with the global trend called "zero residue", i.e. recycling, reusing and/or elimination of waste produced by human activity. This trend reinforces the concept of repurposing environmental liabilities, that is, that which is considered "waste" or "environmental problem" can become a product with income generation and reduction of environmental impacts.

Another global trend, which has also guided modern initiatives of sustainable development, as intended with the microstation is the concept of Circular Economy. This is based on the idea that all components produced within a system, even if expired or disposable, should be recycled and reused by the system itself, thus reducing the losses occurring in Linear Economy systems.

Yet another global trend that is in the conceptual base for the "Microstation" is the concept of Local Economic Development-DEL, which began in Seville-Spain in order to give more competitiveness to the concept of "territory", taking over the world and amassing thousands of supporters. Through DEL, natural resources, notably those in decentralized or local situations, must necessarily integrate development projects.

On the other hand, the biodigestion processes used in the treatment of waste generated by human activities, make biogas available, its use as energy (electric, thermal and vehicular) contributing to the reduction of environmental impacts, namely climate change caused by emissions of greenhouse effect gases to the atmosphere, such as water and soil pollution caused by the accumulation of organic matter in the environments.

The biogas refining and biomethane production process described herein makes it so that biomethane can be used to supply vehicles, a totally renewable and virtually free gas, as it comes from treatment of waste / organic residue.

Refining biogas makes it possible to use the biomethane derived from it, reducing the risk of any environmental impact or damage to vehicles. For this to be possible all material used or waste generated in the process can be recycled and disposed of commercially, this being one of the main advantages in relation to existing processes.

## Claims

1. BIOGAS REFINING AND BIOMETHANE PRODUCTION UNIT **characterized in that** it comprises a biogas compressor (73) with a biogas inlet (74) which compresses the biogas for a CO₂ and H₂S absorption tower (01), a siloxane absorption tower (02), a desorption tower (03), an oxy-catalyst adsorption tower (04), as well as comprising a water tank (05), with a cooling tower (06), a biomethane dryer (07), resulting in biomethane outlet (71), CO₂, H₂S and siloxane outlet (09), wherein the entire refining unit is controlled by an electrical panel (72).

2. BIOGAS REFINING PROCESS using the equipment disclosed in claim 1, **characterized in that** it comprises the following process steps:
1. BIOGAS available for the process is compressed by the biogas compressor (73) according to its composition, with a stabilized pressure;
2. Pressurized BIOGAS is sent to the cooling tower (06) where the temperature of the water used in the process and the temperature of the gas are calibrated and stabilized according to its composition.
3. The gaseous compound (biogas) is sent to the CO₂ and H₂S absorption tower (01), where the removal of CO₂-carbon dioxide and H₂S- Hydrogen Sulfide Gas occurs, using only water as a solvent.
4. BIOMETHANE is sent to the adsorption tower with Oxy-catalyst (04), preventing the passage of any trace of H₂S-Hydrogen Sulfide Gas not detected by the operating sensors.
5. BIOMETHANE is sent to the SILOXANE absorption tower (02) where SILOXANE removal occurs, the SILOXANE absorption tower (02) will only be installed in the unit when BIOMETHANE is contaminated with this agent;
6. BIOMETHANE is sent to a drying system, removal of excess moisture is carried out;
7. BIOMETHANE is pressurized and stored according to all safety standards, in storage tanks (08), and is ready for use in motor vehicles;
8. BIOMETHANE is supplied in motor vehicles using the same technologies available for supply of vehicles with CNG compressed natural gas.

3. PROCESS FOR BIOGAS REFINING AND BIOMETHANE PRODUCTION, according to claim 2, **characterized in that** stabilized pressure of the biogas is in the range of 1 to 35 bar;

4. PROCESS FOR BIOGAS REFINING AND BIOMETHANE PRODUCTION, according to claim 2, **characterized in that** the temperature of the cooling water used in the process and the temperature of the biogas to be stabilized is in the range of 1 to 30 degrees centigrade.

5. PROCESS FOR BIOGAS REFINING AND BIOMETHANE PRODUCTION, according to claim 2, **characterized in that** the contaminants removed are released and processed in other industrial applications.

6. PROCESS FOR BIOGAS REFINING AND BIOMETHANE PRODUCTION, according to claim 2, **characterized in that** the water used in the process is regenerated continuously and its replacement is minimal, caused by natural evaporation.

7. PROCESS FOR BIOGAS REFINING AND BIOMETHANE PRODUCTION, according to claim 2, **characterized in that** all stages of the process are controlled by a set of sensors that are monitored by a supervisory software.

8. PROCESS FOR BIOGAS REFINING AND BIOMETHANE PRODUCTION, according to claim 2, **characterized in that** it processes volumes of 1 to 10 m3 per hour of biogas.

9. PROCESS FOR BIOGAS REFINING AND BIOMETHANE PRODUCTION, according to claim 2, **characterized in that** the output of the biomethane from the CO₂ and H₂S adsorption tower (01) is monitored by sensors that detect the presence of Hydrogen Sulfide Gas and when a level higher than 0% is found, the process is interrupted.
